# EUROPÄISCHE PATENTSCHRIFT

(11) **EP 2 355 813 B1**
(45) Veröffentlichungstag und Bekanntmachung des Hinweises auf die Patenterteilung: **04.11.2015**
(21) Anmeldenummer: 09760502.6
(22) Anmeldetag: 09.11.2009
(51) Int. Cl.: A61K 31/23, A23L 1/30, A61P 3/02

(54) **FETTEMULSION ZUR KÜNSTLICHEN ERNÄHRUNG VON SCHWERKRANKEN INTENSIVPATIENTEN**
FAT EMULSION FOR ARTIFICIALLY FEEDING SERIOUSLY ILL INTENSIVE CARE PATIENTS
ÉMULSION GRASSE POUR L'ALIMENTATION ARTIFICIELLE DE PATIENTS GRAVEMENT MALADES EN SOINS INTENSIFS

(30) Priorität: 18.11.2008 DE 102008057867
(43) Veröffentlichungstag der Anmeldung: 17.08.2011
(73) Patentinhaber: B. Braun Melsungen AG, 34212 Melsungen (DE)
(72) Erfinder: BOLL, Michael, 34212 Melsungen (DE)
(74) Vertreter: Von Kreisler Selting Werner - Partnerschaft von Patentanwälten und Rechtsanwälten mbB
(86) Internationale Anmeldenummer: PCT/EP2009/064839
(87) Internationale Veröffentlichungsnummer: WO 2010/057804

(56) Entgegenhaltungen:
- WO-A-2004/103307
- US-A1- 2003 162 833
- US-B1- 6 740 679
- ROE C R ET AL: "Treatment of cardiomyopathy and rhabdomyolysis in long-chain fat oxidation disorders using an anaplerotic odd-chain triglyceride" JOURNAL OF CLINICAL INVESTIGATION, AMERICAN SOCIETY FOR CLINICAL INVESTIGATION, US, Bd. 110, Nr. 2, 1. Juli 2002 (2002-07-01), Seiten 259-269, XP002995380 ISSN: 0021-9738

## Beschreibung

Die vorliegende Erfindung betrifft eine pharmazeutische Zubereitung für die Prophylaxe und Behandlung der Critical Illness Polyneuropathy (CIP) und der Critical Illness Myopathy (CIM).

Aufgrund von dem unter anderem durch neue Behandlungskonzepte, Klassifikationssysteme und gezielten Interventionen bedingten Fortschritt in der Intensivmedizin der letzten Jahre und Jahrzehnte konnten die Überlebenszeiten schwerstkranker Intensivpatienten deutlich verlängert werden. Dem gegenüber wurden in der Folge jedoch früher seltene oder unbekannte Krankheitsbilder bei diesen Patientengruppe beobachtet. So weisen Intensivpatienten ein besonders hohes Risiko des Entwickelns einer Sepsis auf, wobei dann noch im weiteren Verlauf erhebliche Komplikationen entstehen können. Unter diesen wurden nach systematischen Studien über neurologische und muskuläre Probleme bei Intensivpatienten die Critical Illness Polyneuropathy (CIP) sowie die Critical Illness Myopathy (CIM) ausgemacht. Hierbei handelt es sich in beiden Fällen um eine erworbene Muskelschwäche, und zwar um die primär axonale CIP und die primär muskuläre CIM. Eine klinische Abgrenzung der CIP von der CIM ist überaus schwierig, da in beiden Fällen generalisiserte Paralyse, Muskelschwäche und Entwöhnungsschwierigkeiten vom Respirator die wichtigsten und eben auch gemeinsamen Symptome darstellen und beide Erkrankungen darüber hinaus noch zusammen auftreten können (O. Friedrich, E. Hund, Anaesthesist 2006, 55, 1271-1280). Die Prävalenz von CIP/CIM wird heute bei Patienten mit schwerer Sepsis und multiplen Organversagen von 70 - 80 % angegeben. Auch wenn eine höhere Letalität aufgrund von CIP/CIM alleine wohl nicht besteht, verlängert das Auftreten von CIP/CIM die Intensivtherapie, verzögert die Rehabilitation und führt dadurch zu einer enormen Steigerung der Behandlungs- und volkswirtschaftlichen Kosten. Zudem werden muskuläre Schwäche und schnelle Erschöpfbarkeit von z.B. schwer ARDS-kranken Patienten als wichtigste Ursache für eine eingeschränkte Lebensqualität auch noch nach 12 Monaten nach intensivmedizinischer Behandlung angegeben (M. S. Herridge, A .M. Cheung, C. M. Tansey, N. Engl. J. Med. 2003, 348, 683 - 693). Die genauen Ursachen für CIP/CIM sind derzeit noch unklar und Gegenstand der Forschung in der Intensivmedizin. Als Risikofaktoren werden unter anderem Entzündungsmediatoren, Katabolie, Insulinresistenz, die Anwendung von Kortikosteroiden, erhöhte Glukagonsensitivität, Störung der Energiebereitstellung, die Anwendung von Muskelrelaxantien, oxidativer Stress sowie allgemeine mikrozirkulatorische und/oder Entzündungsreaktionen diskutiert. Aufgrund dessen sind auch spezifische Therapien für CIP/CIM derzeit noch unbekannt. Aus der Literatur ist ersichtlich, dass viele Autoren in der aggressiven Therapie von SIRS und Sepsis (der "Early-goal directed Therapy") den wichtigsten Bestandteil einer CIP/CIM-Therapie sehen. Ferner konnte eine intensivierte Insulintherapie die Inzidenz um 44 % senken, eine weitere Therapieoption wird in der intravenösen Gabe von Immunglobulinen gesehen (M. Alb, S. Hirner, T. Luecke, Anästhesiol. Intensivmed. Notfallmed. Schmerzther. 2007, 4, 250 - 258).

Ein weiterer Hintergrund der vorliegenden Erfindung liegt in dem Gebiet der künstlichen Ernährung von Intensivpatienten durch Fettemulsionen zur intravenösen Anwendung bzw. lipidhaltige Diätetika.

Dabei dienen Fettemulsionen für die parenterale Ernährung der Zufuhr von Fetten in einer intravenös akzeptablen Dosierungsform, wenn eine normale orale Ernährung nicht möglich oder medizinisch kontraindiziert ist. Im Stand der Technik übliche Fettemulsionen werden aus pflanzlichen Ölen wie Distelöl oder Sojaöl hergestellt, in einigen Fällen enthalten sie zusätzlich Triglyceride von mittellangen Fettsäuren (sog. Medium-chain triglycerides (MCT)) und/oder Öle mariner Herkunft (Fischöle meist von Kaltwasserfischen).

So beschreibt DE-OS-37 21 137 Lipidemulsionen für die parenterale Ernährung umfassend Eicosapentaensäure-Triglycerid und/oder Docosahexaensäure-Triglycerid, oder Fischöle, die solche Triglyceride enthalten, genau wie Pflanzenöle, die Omega-6-Fettsäuren enthalten, sowie MCT.

EP 0 120 169 B offenbart synthetische Triglyceride, die am zentralen Kohlenstoffatom des Glycerol-Moleküls eine mehrfach ungesättigte Fettsäure (bevorzugt Eicosapentaensäure) tragen können. Die nach dieser Vorgabe hergestellten Glyceride können zur Ernährung, Ernährungsergänzung oder Arzneimittel für die therapeutische Ernährung verwendet werden.

US-4,526,902 beschreibt Mischungen umfassend 25-75 Gew.-% Eicosapentaensäure und einer Omega-6-Fettsäure, die enteral als Komponente von Pharmazeutika oder fettenthaltenden Nahrungsmitteln wie Butter oder dergleichen verwendet werden.

US 6,740,679 beschreibt *n*-Heptansäure als Energiequelle für Patienten, die an Störungen des Abbaus von langkettigen Fettsäuren leiden.

US 2008/0132571 A1 offenbart Zubereitungen und Methoden für die Behandlung der Katabolismus-Effekte in Patienten, wobei ungeradzahlige Fettsäuren und deren Glyceride zur Steigerung des intrazellulären Verhältnisses von AMP zu ATP und sowie zur Steigerung der Aktivität der AMP-aktivierten Proteinkinase (AMPK) zur Anwendung gebracht werden.

US 2003/0162833 beschreibt eine Methode zur Behandlung von Patienten mit Herz-Kreislauf-Erkrankungen, bei der den Patienten eine Fettsäure mit sieben Kohlenstoffatomen verabreicht wird.

US 6,740,679 beschreibt die Verwendung einer C7-Fettsäure, insbesondere n-Heptansäure, als Nahrungsergänzung.

WO 2004/103307 offenbart die Behandlung von Medium-Chain-Acyl-CoA-Dehydrogenase (MCAD)-Mangel mit C5- oder C15-Fettsäurequellen.

Wirksame Behandlungsmethoden von CIP und CIM sind kaum bekannt. Darüber hinaus sind wirksame nutritive Methoden zur Behandlung der Sepsis bzw. der Folgekomplikationen einer Intensivtherapie wie der CIP und/oder der CIM zur Zeit kaum bekannt.

Es besteht somit ein Bedarf an Stoffen und Zubereitungen für die künstliche Ernährung sowie der begleitenden nutritiven Behandlung von Intensivpatienten und es besteht ein dringender Bedarf an Zubereitungen und Methoden zur Prophylaxe und Therapie von CIP und/oder CIM. Vor diesem Hintergrund bestand die Aufgabe der vorliegenden Erfindung darin, eine pharmazeutische Zubereitung zur begleitenden nutritiven Behandlung kritisch kranker, z. B. septischer Intensivpatienten und zur Prophylaxe und Therapie von Folgekomplikationen der Intensivtherapie wie der CIP und/oder CIM bereit zu stellen.

Überraschenderweise wurde gefunden, dass durch Zufuhr einer Fettemulsion, die Triglyceride mit Fettsäureresten aufweist, die eine ungerade Zahl von Kohlenstoffatomen aufweisen, die Häufigkeit des Auftretens der genannten Komplikationen reduziert oder der Schweregrad der Erkrankung gemildert bzw. ihr Verlauf abgekürzt werden können.

Ein Gegenstand der vorliegenden Erfindung ist somit eine pharmazeutische Zubereitung zur Prophylaxe oder Behandlung von CIP und/oder CIM, umfassend eine Fettemulsion mit mindestens einem Triglycerid (A) der Formel (I), 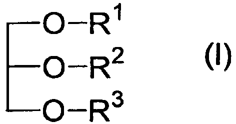 wobei mindestens einer der Reste R¹, R² oder R³ n-Heptanoyl ist.

Gemäß der vorliegenden Erfindung besitzen die Alkanoylreste eine Kettenlänge von 5 bis 15 Kohlenstoffatomen. Bevorzugt handelt es sich um die Alkanoyle, die von einer oder mehreren der folgenden Fettsäuren ausgewählt aus der Gruppe bestehend aus Pentansäure (C5:0, n-Valeriansäure), Heptansäure (C7:0, Önanthsäure), Nonansäure (C9:0, Pelargonsäure), Undecansäure (C11:0, Undecylsäure), Tridecansäure (C13:0, Tridecylsäure) und Pentadecansäure (C15:0, Pentadecylsäure) abgeleitet sind.

Erfindungsgemäß ist dabei, dass mindestens einer der Reste R¹, R² oder R³ im Triglycerid (A) *n*-Heptanoyl ist, also ein Triglycerid das aus einem veresterten Glycerin besteht, wobei mindestens eine, vorzugsweise mindestens zwei Hydroxygruppen mit Heptansäure verestert sind.

Insbesondere ist das Triglycerid (A) Triheptanoin, also ein Glycerin, bei dem die drei Hydroxygruppen mit n-Heptansäure verestert sind.

Die Synthese des Triglycerid (A) ist dem Fachmann geläufig. Die dazu erforderlichen ungeradzahligen Fettsäuren (Pentan-, Heptan-, Nonan-, Undecan-, Tridecan- und Pentadecansäure) sind kommerziell erhältlich, z.B. bei Sigma Chemicals Co.. Ebenso gibt es zahlreiche kommerzielle Bezugsquellen verschiedener Varianten des Triglycerids (A) als solchem: Beispielsweise kann Triheptanoin von der Condea Chemie GmbH (Witten, Deutschland) als Spezialöl 107 bezogen werden. Trinonanoin, Triundecanoin oder Tripentadecanoin sind z. B. durch die Chemos GmbH (Regenstauf, Deutschland) lieferbar.

Das Triglycerid (A) kann neben dem erfindungsgemäß wesentlichen Fettsäurerest n-Heptanoyl auch weitere geradzahlige Fettsäurereste aufweisen. Hier sind in der erfindungsgemäßen pharmazeutischen Zubereitung Fettemulsionen bevorzugt, die das Triglycerid (A) aufweisen, bei denen mindestens ein Fettsäurerest von omega-3 und/oder omega-6 Fettsäuren herrührt. Omega-3 und omega-6 Fettsäuren sind biologisch wichtige Bausteine/Nährstoffe, die der menschliche Organismus selbst nicht vollständig herstellen kann, und die als Präkursoren für Prostaglandine, Eicosanoide und Strukturbestandteile von Zellmembranen fungieren. Als Quelle für omega-6 Fettsäuren dienen verschiedene Öle pflanzlichen Ursprungs, darunter Sojabohnenöl und Safflordistelöl, deren Verwendung für die Herstellung intravenöser Fettemulsionen zum Stand der Technik gehört. Ebenfalls zum Stand der Technik gehört die Verarbeitung von Ölen marinen Ursprungs ("Fischöl") als Quelle für omega-3 Fettsäuren in intravenösen Fettemulsionen (EP-A-0298293), wobei hochgereinigte Fischölkonzentrate bevorzugt werden, deren Gewinnung aus Kaltwasserfischen wie Lachs, Hering, Sardinen oder Makrelen erfolgt. Ihr Gehalt an omega-3 Fettsäuren liegt vorzugsweise bei 40 % oder darüber.

Weiter bevorzugt ist Triglycerid (A) bei dem mindestens ein Fettsäurerest ausgewählt ist aus der Gruppe bestehend aus mittelkettigen Fettsäuren (z. B. Caprylsäure C8:0, Caprinsäure C10:0, Laurinsäure C12:0), langkettigen gesättigten Fettsäuren (z. B. Myristinsäure C14:0, Palmitinsäure C16:0, Stearinsäure C18:0), einfach ungesättigten Fettsäuren (Palmitoleinsäure C16:1, Ölsäure C18:1), mehrfach ungesättigten Fettsäuren vom omega-3 und omega-6-Typ, z.B. Eicosapentaensäure (EPA, C20:5 omega-3), Docosahexaensäure (DHA, C22:6 omega-3), Linolsäure (LA, C18:2 omega-6) oder Gamma-Linolensäure (GLA, C18:3 omega-6).

In einer weiteren bevorzugten Ausführungsform der vorliegenden Erfindung liegt das Triglycerid (A) als ein randomisiertes strukturiertes Lipid mit einer zufälligen Verteilung der Alkanoyl-Reste mit ungeradzahliger Kohlenstoffzahl und der Alkanoyl-Reste mit geradzahliger Kohlenstoffzahl in den Positionen sn-1, sn-2 und sn-3 des Triglyceridmoleküls (A) vor. Alternativ und speziell bevorzugt liegt das Triglycerid (A) als ein chemisch definiertes strukturiertes Lipid vor, das heißt, es liegt eine chemisch definierte Verteilung der Alkanoyl-Reste mit ungeradzahliger Kohlenstoffzahl in den Positionen sn-1 und sn-3 und der Alkanoyl-Reste mit geradzahliger Kohlenstoffzahl an Position sn-2 des Triglyceridmoleküls vor.

Für den Fall, dass die einzusetzenden Fettemulsionen ganz oder teilweise chemisch definierte oder randomisierte strukturierte Lipide enthalten, dienen bevorzugt pflanzliche Öle als Quelle für omega-6 Fettsäuren bzw. Fischöl als Quelle für omega-3 Fettsäuren zur Herstellung der strukturierten Lipide. Randomisierte strukturierte Triglyceride sind beispielsweise erhältlich durch chemische Umesterung eines Gemischs aus einem gewünschten Pflanzen- und/oder Fischöl mit einem Triglycerid aus ungeradzahligen Fettsäuren mit einer Kettenlänge von 5 bis 15. Im Fall chemisch definierter strukturierter Lipide erfolgt die Umesterung auf enzymatischen Weg ausgehend von den gleichen Grundstoffen.

In einer weiteren bevorzugten Ausführungsform der vorliegenden Erfindung unsfasst die erfindungsgemäße pharmazeutische Zubereitung eine Fettemulsion, die mindestens ein zusätzliches Triglycerid (B) umfasst, das von (A) verschieden ist.

Bevorzugt umfasst das Triglycerid (B) Triglyceride marinen oder pflanzlichen Ursprungs. Da die Gewinnung reiner omega-3 oder omega-6 Fettsäuren aus Fischöl bzw. Pflanzenölen oder die chemische Synthese dieser Fettsäuren einerseits aufwendig und kostspielig ist, während andererseits die genannten Öle marinen oder pflanzlichen Ursprungs einen hohen Gehalt an den entsprechenden Fettsäuren aufweisen, ist es entsprechend der vorliegenden Erfindung nicht erforderlich, omega-3 oder oder omega-6 Fettsäuren bzw. omega-3 oder omega-6 Fettsäuren enthaltende Triglyceride aus diesen Ölen zu isolieren, sondern die Öle können als solche zur Herstellung der erfindungsgemäß einzusetzenden Fettemulsionen verwendet werden.

Durch den Einsatz von Fischöl, besonders aber von Sojabohnen- oder Safflordistelöl, werden automatisch auch langkettige gesättigte Fettsäuren bereitgestellt wie deren oben erwähnte Vertreter Myristin-, Palmitin- und Stearinsäure, desgleichen die einfach ungesättigte Ölsäure, die sowohl in marinen Ölen und - in besonders hoher Konzentration - in Olivenöl enthalten ist.

Mittelkettige Fettsäuren bzw. Triglyceride sind in halbsynthetischem MCT-Öl (Miglyolöl) enthalten und zwar zu über 90 % (bezogen auf den Gesamtfettsäurengehalt) als Capryl- und Caprinsäure. In dieser Form sind sie besonders geeignet, als Triglycerid (B) in die erfindungsgemäß einzusetzende Fettemulsion eingesetzt zu werden.

In einer bevorzugten Ausführungsform umfasst die erfindungsgemäße pharmazeutische Zubereitung eine Emulsion, die neben Triglycerid (A) mindestens ein weiteres Triglycerid (B) umfasst, das mindestens einen Fettsäurerest, der ausgewählt ist aus der Gruppe bestehend aus mittelkettigen Fettsäuren (z. B. Caprylsäure C8:0, Caprinsäure C10:0, Laurinsäure C12:0), langkettigen gesättigten Fettsäuren (z. B. Myristinsäure C14:0, Palmitinsäure C16:0, Stearinsäure C18:0), einfach ungesättigten Fettsäuren (Palmitoleinsäure C16:1, Ölsäure C18:1), mehrfach ungesättigten Fettsäuren vom omega-3 und omega-6-Typ, z.B. Eicosapentaensäure (EPA, C20:5 omega-3), Docosahexaensäure (DHA, C22:6 omega-3), Linolsäure (LA, C18:2 omega-6), Gamma-Linolensäure (GLA, C18:3 omega-6), enthält.

In einer weiteren bevorzugten Ausführungsform der vorliegenden Erfindung beträgt die Menge an Triglycerid (A) 50 bis 80 Gew.-%, weiter bevorzugt 60 bis 70 Gew.-%, bezogen auf das Gesamtgewicht aller Triglyceride in der Emulsion.

Bevorzugt weist die erfindungsgemäße pharmazeutische Zubereitung Triglyceride in einer Menge von 5 bis 30 Gew. %, weiter bevorzugt von 10 bis 20 Gew.-%, bezogen auf die gesamte pharmazeutische Zubereitung, auf.

Die Fettemulsion der erfindungsgemäßen pharmazeutischen Zubereitung enthält neben dem Triglycerid (A) und gegebenfalls anderen Triglyceriden/Lipiden vorteilhafterweise Wasser für Injektionszwecke sowie weitere Hilfsstoffe und Additive, wie sie dem Stand der Technik bei der Herstellung intravenöser Fettemulsionen entsprechen, z.B. Emulgatoren, Co-Emulgatoren, Stabilisatoren und geeignete Stoffe zur Einstellung der Isotonie.

Als Emulgatoren werden physiologisch gut verträgliche Emulgatoren verwendet, wie z. B. Phospholipide tierischer oder pflanzlicher Herkunft. Bevorzugt werden gereinigte Lezithine, so z. B. Sojabohnenlezithin oder Eilezithin oder daraus gewonnene Partialfraktionen, eingesetzt. Der Phospholipidgehalt in der erfindungsgemäß einzusetzenden Emulsion beträgt bevorzugt 0.4 bis 2.0 Gew.-%, vorzugsweise 0.6 % bis 1.5 % Gew.-%, jeweils bezogen auf das Gesamtgewicht der Emulsion.

Weiterhin können Co-Emulgatoren eingesetzt werden, z. B. die Alkalisalze von langkettigen Fettsäuren (wie Natrium-Stearat, Natrium-Oleat usw.) oder - als alleinige Co-Emulgatoren oder in Kombination mit anderen - Cholesterin bzw. Cholesterinester (z. B. Cholesterin-Acetat). Wenn Alkalisalze langkettiger Fettsäuren als Co-Emulgatoren Verwendung finden, beträgt ihre Konzentration ebenso wie im Fall der Verwendung von Cholesterin bzw. Cholesterinestern allein oder in Verbindung mit anderen Co-Emulgatoren jeweils 0.01 Gew.-% bis 0.1 Gew.- %, vorzugsweise 0.02 bis 0.04 Gew.-%, jeweils bezogen auf das Gesamtgewicht der Emulsion.

Die Fettemulsion der erfindungsgemäßen pharmazeutischen Zubereitung kann, speziell wenn sie vielfach ungesättigte Fettsäuren wie omega-3 oder omega-6 Fettsäuren enthält, mit Antioxidantien angereichert sein, die vor der Bildung von unerwünschten Peroxiden schützen. Als Antioxidantien kommen vor allem Vitamin E (alpha-, beta- oder gamma-Tokopherol) und Vitamin C (z. B. als Ascorbylpalmitat) in Betracht, wobei die Vitamine E und C bzw. ihre Isomere oder Derivate entweder allein oder in Kombination vorliegen können. Abhängig vom Gehalt langkettiger, speziell mehrfach ungesättigter Fette in der erfindungsgemäßen Zubereitung beträgt ihr Gewichtsanteil zwischen 0.002 und 0.03 % (alpha-Tokopherol) bzw. 0.001 und 0.015 % (Ascorbylpalmitat), jeweils bezogen auf das Gesamtgewicht der erfindungsgemäß einzusetzenden Emulsion.

In einer speziellen Ausführungsform enthält die Fettemulsion, die erfindungsgemäß in die die erfindungsgemäße pharmazeutische Zubereitung einzusetzen ist, außerdem L-Carnitin in einer Menge von vorzugsweise 0.01 bis 0.1 Gew.- %, jeweils bezogen auf das Gesamtgewicht der Emulsion.

In einer weiteren Ausführungsform kann die erfindungsgemäße Zubereitung außerdem die Vitamine Biotin und/oder Cobalamin enthalten: Ihre Konzentrationen betragen 1 - 10 mg Biotin bzw. 0.1 - 1 mg Cobalamin pro 100 g Lipidanteil der Zubereitung.

Die Isotonisierung der Fettemulsion erfolgt bevorzugt mit Hilfe von Polyolen wie Glycerol, Xylitol oder Sorbitol, die in einer Menge von vorzugsweise 2 bis 3 Gew.-% jeweils bezogen auf das Gesamtgewicht der Emulsion, zur Anwendung kommen. Als bevorzugtes Isotonisierungsmittel dient Glycerol.

Das pharmazeutische Mittel gemäß der vorliegenden Erfindung wird in einer pharmazeutisch wirksamen Menge verabreicht. Bevorzugt liegt die pharmazeutisch wirksame Dosis, d.h. diejenige Menge an Triglycerid (A), die mit der erfindungsgemäßen pharmazeutischen Zubereitung zuzuführen ist, um die Komplikationen schwerer, z. B. septischer Erkrankungen und ihrer intensivmedizinischen Behandlung zu vermeiden, ihre Intensität zu mildern und Dauer zu verkürzen, bei 1 bis 2 g pro kg Körpergewicht und Tag. Die Zufuhr erfolgt vorzugsweise kontinuierlich über 24 Stunden/Tag, kann jedoch auch auf mehrere Portionen verteilt werden, wobei eine Infusionsgeschwindigkeit von 0.25 g Fett pro kg Körpergewicht und Stunde nicht überschritten werden sollte. Im allgemeinen ist eine mehrtägige mittel- bis langfristige Verabreichung erforderlich, um die erfindungsgemäße Wirkung zu erzielen.

Die Anwendung der erfindungsgemäßen pharmazeutischen Zubereitung erfolgt als Bestandteil einer vollständig parenteralen oder kombiniert parenteralen/enteralen Ernährung, wie sie bei schwerkranken z. B. septischen Intensivpatienten mit kompliziertem Verlauf bzw. manifester oder drohender muskulärer Neuropathie angezeigt ist. Dabei ist der hohe Energiegehalt der erfindungsgemäßen Emulsion bei der Gesamtkalorienzufuhr mit der parenteralen oder kombiniert enteralen/parenteralen Ernährung zu berücksichtigen. Je nach der zur Anwendung kommenden Ausführungsform der erfindungsgemäßen Fettemulsion - mit oder ohne Anteil an essentiellen Fettsäuren (omega-3 bzw. omega-6) - müssen letzgenannte nicht zusätzlich substituiert werden. Speziell durch die besonders bevorzugte Anwesenheit von omega-3 Fettsäureresten in der Emulsion und deren antientzündliche Eigenschaften lassen sich synergistische Wirkungen erzielen und der Heilungsprozeß zusätzlich begünstigen.

Ein weiterer Gegenstand ist eine isotone Fettemulsion umfassend ein Triglycerid (A) der Formel (I), 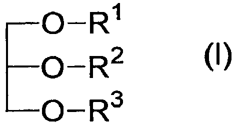 wobei mindestens einer der Reste R¹, R² oder R³ ein Alkanoyl-Rest mit einer ungeraden Zahl von 5 bis 15 Kohlenstoffatomen ist und die mindestens ein zusätzliches Triglycerid (B) umfasst, das von (A) verschieden ist und das mindestens einen Fettsäurerest aufweist, der ausgewählt ist aus der Gruppe bestehend aus mittelkettigen Fettsäuren, umfassend Caprylsäure, Caprinsäure oder Laurinsäure; langkettigen gesättigter Fettsäuren, umfassend Myristinsäure, Palmitinsäure oder Stearinsäure; einfach ungesättigten Fettsäuren, umfassend Palmitoleinsäure oder Ölsäure; und mehrfach ungesättigten Fettsäuren vom omega-3- und omega-6-Typ, umfassend Eicosopentaensäure, Docosahexaensäure, Linolsäure und Gamma-Linolensäure.

Weitere bevorzugte Ausgestaltungen der isotonen Fettemulsion entsprechen der Fettemulsion, die in die erfindungsgemäße pharmazeutische Zubereitung einzusetzen ist.

Ein weiterer Gegenstand ist die Verwendung der isotonen Fettemulsion als Diätetikum. Die isotone Fettemulsion wird hierzu bevorzugt zur enteralen Ernährung verwendet.

Ein weiterer Gegenstand ist ein Diätetikum, umfassend mindestens ein Triglycerid (A) der Formel (I). Bevorzugt umfasst das Diätetikum die isotone Fettemulsion.

Ein weiterer Gegenstand ist die Verwendung der isotonen Fettemulsion oder einer Fettemulsion mit mindestens einem Triglycerid (A) der Formel (I) oder der erfindungsgemäßen pharmazeutischen Zubereitung zur künstlichen Ernährung septischer Intensivpatienten und/oder zur parenteralen Ernährung.

Ein weiterer Gegenstand ist ein Arzneimittel, umfassend mindestens ein Triglycerid (A) der Formel (I), 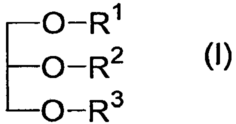 wobei unabhängig voneinander mindestens einer der Reste R¹, R² oder R³ ein Alkanoyl-Rest mit einer ungeraden Zahl von 5 bis 15 Kohlenstoffatomen ist, sowie mindestens ein weiteres Triglycerid (B), das von (A) verschieden ist. Bevorzugt umfasst das Arzneimittel die erfindungsgemäße isotone Fettemulsion.

Zur Herstellung der erfindungsgemäßen pharmazeutischen Zubereitung sowie der isotonen Fettemulsion als auch des Arzneimittels werden die in der nachstehenden Tabelle aufgeführten lipophilen Komponenten 1 - 9 (soweit je nach Herstellungsbeispiel benötigt) grob gemischt und mit Hilfe eines Ultra-Turrax Homogenisators dispergiert. Danach werden die hydrophilen Komponenten 10 - 13 zugefügt, wobei Natrium-Oleat bzw. -Stearat und NaOH als wässrige Lösungen verwendet und der pH der Ansatzmischung mit Hilfe der zuletzt genannten Komponenten auf einen Wert von 8.0 - 9.0 eingestellt werden. Die eigentliche Homogenisation des Ansatzes erfolgt im Anschluß daran in einem Hochdruckhomogenisator bei Drucken von ca. 400 kg/cm². Die fertige Emulsion wird in geeignete Glas- oder Kunststoffbehälter abgefüllt und nach den für Parenteralia üblichen Verfahren hitzesterilisiert. Dabei ergibt sich eine sterile, pyrogenfreie und stabile Fettemulsion, die eine mittlere Tröpfchengröße von weniger als 0.5 µm aufweist und eine Haltbarkeit von wenigstens 24 Monaten bei Raumtemperatur besitzt.

### Beispiele

| | | Beispiel 1 | Beispiel 2 | Beispiel 3 | Beispiel 4 | Beispiel 5 | Vgl.-Beispiel 6 | Beispiel 7 |
|---|---|---|---|---|---|---|---|---|
| 1 | Triheptanoin | 800 g | 800 g | 1400 g | 1000 g | 600 g | - | 1500 g |
| 2 | Mittelkettige Triglyceride¹⁾ | - | - | - | 400 g | - | - | - |
| 3 | Sojabohnenöl | 200 g | - | - | 300 g | - | - | 1000 g |
| 4 | Safflordistelöl | - | - | 300 g | - | 200 g | - | - |
| 5 | Fischölkonzentr. | - | 200 g | 300 g | 300 g | 200 g | - | 500 g |
| 6 | Struktur. Lipid²⁾ | - | - | - | - | - | 1000 | - |
| 7 | Phospholipide | 80 g | 80 g | 120 g | 120 g | 80 g | 80 g | 150 g |
| 8 | α-Tocopherol | 500 mg | 500 mg | 1500 mg | 1500 mg | 1000 mg | 50 mg | 2000 mg |
| 9 | AscorbylPalmitat | 200 mg | 200 mg | 600 mg | 300 mg | 400 mg | 200 mg | 1000 mg |
| 10 | Natrium-Oleat | 3.0 g | 3.0 g | 3.0 g | 3.0 g | 3.0 g | 3.0 g | 3.0 g |
| 11 | Glycerol | 25 | 25 | 22.5 | 22.5 | 25 | 25 | 20 |
| 12 | NaOH | ad pH 8-9 | ad pH 8-9 | ad pH 8-9 | ad pH 8-9 | ad pH 8-9 | ad pH 8-9 | ad pH 8-9 |
| 13 | Wasser für Injektionszwecke | ad 10 Liter | ad 10 Liter | ad 10 Liter | ad 10 Liter | ad 10 Liter | ad 10 Liter | ad 10 Liter |

| | | | | | | | | |
|---|---|---|---|---|---|---|---|---|
| ¹⁾ Für den Anteil der mittelkettigen Triglyceride (Caprylsäure/Caprinsäure Triglyceride) wurde eine handelsübliche Mischung (Miglyol 812, Sasol Germany GmbH, Witten, Deutschland) verwendet. ²⁾ Als strukturiertes Lipid wurde das Triglycerid (A), bestehend aus Glycerin, welches mit Heptansäure in den Positionen sn-1 und sn-3 und Eicosapentaensäure ((EPA), C2O:5 Omega- 3) in der Position sn-2 des Triglycerids verestert ist, eingesetzt. | | | | | | | | |

## Patentansprüche

1. Pharmazeutische Zubereitung zur Verwendung in der Prophylaxe oder Behandlung von CIP und/oder CIM, umfassend eine Fettemulsion mit mindestens einem Triglycerid (A) der Formel (I), 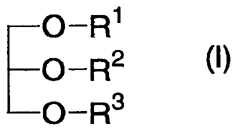 wobei mindestens einer der Reste R¹, R² oder R³ n-Heptanoyl ist.

2. Pharmazeutische Zubereitung zur Verwendung in der Prophylaxe oder Behandlung von CIP und/oder CIM gemäß Anspruch 1, **dadurch gekennzeichnet, dass** die Fettemulsion mindestens ein zusätzliches Triglycerid (B) umfasst, das von (A) verschieden ist und das mindestens einen Fettsäurerest aufweist, der ausgewählt ist aus der Gruppe bestehend aus mittelkettiger Fettsäuren, umfassend Caprylsäure, Caprinsäure oder Laurinsäure; langkettiger gesättigter Fettsäuren, umfassend Myristinsäure, Palmitinsäure oder Stearinsäure; einfach ungesättigter Fettsäuren, umfassend Palmitoleinsäure oder Ölsäure; und mehrfach ungesättigter Fettsäuren vom omega-3- und omega-6-Typ, umfassend Eicosopentaensäure, Docosahexaensäure, Linolsäure und Gamma-Linolensäure.

3. Pharmazeutische Zubereitung zur Verwendung in der Prophylaxe oder Behandlung von CIP und/oder CIM gemäß irgendeinem der Ansprüche 1 und 2, **dadurch gekennzeichnet, dass** das Triglycerid (A) Triheptanoin ist.

4. Pharmazeutische Zubereitung zur Verwendung in der Prophylaxe oder Behandlung von CIP und/oder CIM gemäß irgendeinem der Ansprüche 1 bis 3, **dadurch gekennzeichnet, dass** die Menge an Triglyceriden 5 bis 30 Gew. %, bezogen auf die gesamte pharmazeutische Zubereitung, beträgt.

5. Pharmazeutische Zubereitung zur Verwendung in der Prophylaxe oder Behandlung von CIP und/oder CIM gemäß irgendeinem der Ansprüche 1 bis 4, **dadurch gekennzeichnet, dass** die Menge an Triglycerid (A) 50 bis 80 Gew.-%, bezogen auf das Gesamtgewicht aller Triglyceride in der Emulsion, beträgt.

6. Pharmazeutische Zubereitung zur Verwendung in der Prophylaxe oder Behandlung von CIP und/oder CIM gemäß irgendeinem der Ansprüche 1 bis 5, **dadurch gekennzeichnet, dass** die Menge an Triglycerid (A) 60 bis 70 Gew.-%, bezogen auf das Gesamtgewicht aller Triglyceride in der Emulsion, beträgt.

## Claims

1. A pharmaceutical formulation for use in the prophylaxis or treatment of CIP and/or CIM comprising a fat emulsion containing at least one triglyceride (A) of formula (I): 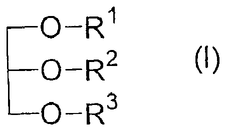 wherein at least one of radicals R¹, R² or R³ is n-heptanoyl.

2. The pharmaceutical formulation for use in the prophylaxis or treatment of CIP and/or CIM according to claim 1, **characterized in that** said fat emulsion comprises at least one additional triglyceride (B) different from (A) and having at least one fatty acid residue selected from the group consisting of medium-chain fatty acids including caprylic acid, capric acid or lauric acid, long-chain saturated fatty acids including myristic acid, palmitic acid or stearic acid, monounsaturated fatty acids including palmitoleic acid or oleic acid, and polyunsaturated fatty acids of omega-3 and omega-6 type including eicosapentaenic acid, docosahexaenic acid, linolic acid and gamma-linolenic acid.

3. The pharmaceutical formulation for use in the prophylaxis or treatment of CIP and/or CIM according to either of claims 1 and 2, **characterized in that** said triglyceride (A) is triheptanoin.

4. The pharmaceutical formulation for use in the prophylaxis or treatment of CIP and/or CIM according to any of claims 1 to 3, **characterized in that** said the amount of triglycerides is from 5 to 30% by weight, based on the total pharmaceutical formulation.

5. The pharmaceutical formulation for use in the prophylaxis or treatment of CIP and/or CIM according to any of claims 1 to 4, **characterized in that** the amount of triglyceride (A) is from 50 to 80% by weight, based on the total weight of all triglycerides in the emulsion.

6. The pharmaceutical formulation for use in the prophylaxis or treatment of CIP and/or CIM according to any of claims 1 to 5, **characterized in that** the amount of triglyceride (A) is from 60 to 70% by weight, based on the total weight of all triglycerides in the emulsion.

## Revendications

1. Formulation pharmaceutique destinée à être utilisée dans la prophylaxie ou le traitement de la CIP et/ou de la CIM, comprenant une émulsion de graisse avec au moins un triglycéride (A) de la formule (I), 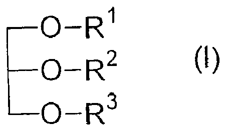 dans laquelle au moins un des radicaux R¹, R² ou R³ est le n-heptanoyle.

2. Formulation pharmaceutique destinée à être utilisée dans la prophylaxie ou le traitement de la CIP et/ou de la CIM selon la revendication 1, **caractérisée en ce que** ladite émulsion de graisse comprend au moins un triglycéride additionnel (B) autre qu'(A) possédant au moins un reste d'acide gras choisi dans le groupe consistant en acides gras à chaînes moyennes, y compris l'acide caprylique, l'acide caprique ou l'acide laurique, acides gras saturés à chaînes longues, y compris l'acide myristique, l'acide palmitique ou l'acide stéarique, acides gras mono-insaturés y compris l'acide palmitoléique ou l'acide oléique, et acides gras poly-insaturés de type oméga-3 et oméga-6, y compris l'acide eicosapentaénoïque, l'acide docosahexaénoïque, l'acide linolique et l'acide gamma-linolénique.

3. Formulation pharmaceutique destinée à être utilisée dans la prophylaxie ou le traitement de la CIP et/ou de la CIM selon l'une des revendications 1 et 2, **caractérisée en ce que** ledit triglycéride (A) est la triheptanoïne.

4. Formulation pharmaceutique destinée à être utilisée dans la prophylaxie ou le traitement de la CIP et/ou de la CIM selon l'une quelconque des revendications 1 à 3, **caractérisée en ce que** la quantité de triglycérides est de 5 à 30 % en poids, par rapport à la quantité totale de la formulation pharmaceutique.

5. Formulation pharmaceutique destinée à être utilisée dans la prophylaxie ou le traitement de la CIP et/ou de la CIM selon l'une quelconque des revendications 1 à 4, **caractérisée en ce que** la quantité du triglycéride (A) est de 50 à 80 % en poids, par rapport au poids total de tous les triglycérides dans l'émulsion.

6. Formulation pharmaceutique destinée à être utilisée dans la prophylaxie ou le traitement de la CIP et/ou de la CIM selon l'une quelconque des revendications 1 à 5, **caractérisée en ce que** la quantité du triglycéride (A) est de 60 à 70 % en poids, par rapport au poids total de tous les triglycérides dans l'émulsion.
